# EUROPEAN PATENT APPLICATION

(11) **EP 4 681 663 A1**
(43) Date of publication of application: **21.01.2026**
(21) Application number: 24774788.4
(22) Date of filing: 13.03.2024
(51) Int. Cl.: A61B 17/435

(54) **EMBRYO TRANSPLANTATION DEVICE**

(30) Priority: 17.03.2023 JP 2023042535
(71) Applicant: INSTITUTE OF SCIENCE TOKYO, Tokyo 152-8550 (JP)
(72) Inventor: FUKUOKA, Muneaki, Tokyo 101-0062 (JP); IKEUCHI, Masashi, Tokyo 152-8550 (JP)
(74) Representative: Nederlandsch Octrooibureau
(86) International application number: PCT/JP2024/009757
(87) International publication number: WO 2024/195650

(57) **Abstract**

An embryo transfer device including a proximal end portion forming a side of a proximal end, an embryo accommodation portion, and two or more expandable frame parts, wherein the two or more expandable frame parts are configured to become a state of being close to each other due to elastic deformation when disposed in a tube, and to expand in a direction orthogonal to an X axis passing through the proximal end portion when released from the tube to be disposed in a uterine cavity.

## Description

### {Technical Field}

The present disclosure relates to embryo transfer devices.

### {Background Art}

Contraceptive devices have hitherto been known as devices that are disposed in uterine cavities. For example, see PTL 1. Furthermore, embryo transfer devices that utilize magnetic force have been known as devices that are disposed in uterine cavities. For example, see PTL 2. Furthermore, implanting an embryo into a uterus by using a linear instrument has been known. For example, see PTL 3.

### {Citation List}

### {Patent Literature}

{PTL 1}
   Japanese Patent Application, Publication No. 2014-523780
{PTL 2}
   PCT International Publication No. WO 2018/135496
{PTL 3}
   Japanese Patent Application, Publication No. 2009-508642

### {Summary of Invention}

### {Technical Problem}

Various conditions contribute to improving the probability of implantation, including the force applied from the device to the uterus, damage to the uterine cervix, the uterine cavity, etc. at the time of insertion/withdrawal of the device, and the status of indwelling of the embryo for the purpose of implantation. It is hoped that practical and effective devices that can improve the probability of implantation be realized.

### {Solution to Problem}

A first aspect is an embryo transfer device that has a proximal end and a distal end and that is inserted into a uterine cavity from a side of the distal end by using a tube inserted into a uterine cervix, the embryo transfer device including: a proximal end portion forming a side of the proximal end; an embryo accommodation portion that is disposed on the side of the distal end and that allows an embryo to be accommodated therein; and two or more expandable frame parts that connect the proximal end portion and the embryo accommodation portion with each other, wherein the two or more expandable frame parts are configured to become a state of being close to each other due to elastic deformation when disposed in the tube, and to expand in a direction along a Y axis due to a restoring force of the elastic deformation when released from the tube to be disposed in the uterine cavity, the Y axis being orthogonal to an X axis passing through the proximal end portion and the embryo accommodation portion.

A second aspect of the present invention is an embryo transfer device that has a proximal end and a distal end and that is inserted into a uterine cavity from a side of the distal end by using a tube inserted into a uterine cervix, the embryo transfer device including: an embryo accommodation portion that allows an embryo to be accommodated therein; and two or more expandable frame parts, wherein the two or more expandable frame parts become a state of being close to each other due to elastic deformation when disposed in the tube, and expand in a direction along a Y axis due to a restoring force of the elastic deformation when released from the tube to be disposed in the uterine cavity, the Y axis being orthogonal to an X axis passing through the proximal end and the distal end, and wherein the embryo accommodation portion is opened in a direction along a Z axis that is orthogonal to the X axis and the Y axis.

A third aspect of the present invention is an embryo transfer device that is inserted into a uterine cavity by using a tube inserted into a uterine cervix, the embryo transfer device including: an embryo accommodation portion that allows an embryo to be accommodated therein; and a frame part that has a proximal end and a distal end and that is connected to the embryo accommodation portion, wherein an expandable part having one end fixed to the frame part or the frame part becomes an accommodated state following a shape of an interior of the tube when disposed in the tube, and is expanded mainly in a direction along a Y axis when released from the tube to be disposed in the uterine cavity, the direction along the Y axis being a direction orthogonal to an X axis passing through the proximal end and the distal end, and wherein the embryo accommodation portion is opened in a direction along a Z axis that is orthogonal to the X axis and the Y axis.

### {Brief Description of Drawings}

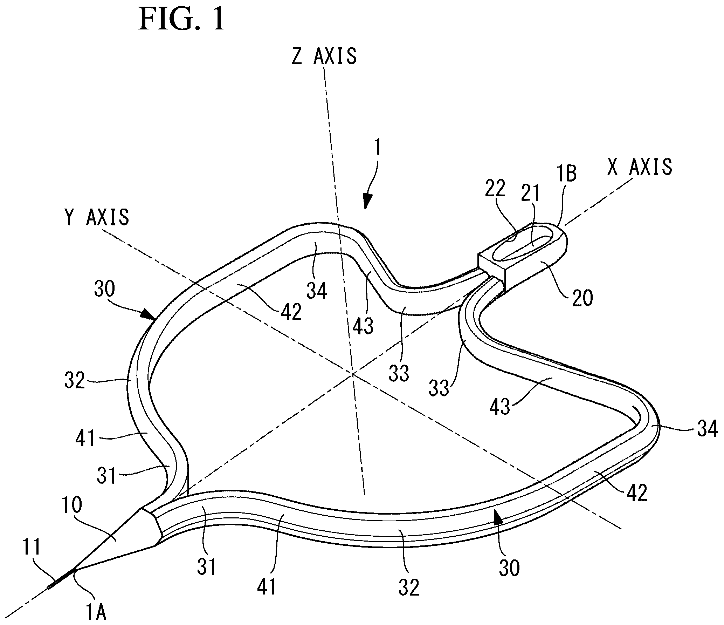

{Fig. 1}
   Fig. 1 is a perspective view of an embryo transfer device of a first embodiment.
{Fig. 2}
   Fig. 2 is a plan view of the embryo transfer device in the first embodiment.
{Fig. 3}
   Fig. 3 is a side view of the embryo transfer device in the first embodiment.
{Fig. 4}
   Fig. 4 is a plan view in a state in which the embryo transfer device in the first embodiment is used.
{Fig. 5}
   Fig. 5 is a sectional view taken along V-V in Fig. 4.
{Fig. 6}
   Fig. 6 is an X-axis sectional view of an embryo accommodation portion of the embryo transfer device in the first embodiment.
{Fig. 7}
   Fig. 7 is a plan view in a state in which the embryo transfer device in the first embodiment is used.
{Fig. 8}
   Fig. 8 is a plan view in a state in which the embryo transfer device in the first embodiment is used.
{Fig. 9}
   Fig. 9 is a plan view showing a first modified embodiment of the embryo transfer device in the first embodiment.
{Fig. 10}
   Fig. 10 is a plan view showing a second modified embodiment of the embryo transfer device in the first embodiment.
{Fig. 11}
   Fig. 11 is a plan view showing a third modified embodiment of the embryo transfer device in the first embodiment.
{Fig. 12}
   Fig. 12 is a plan view showing a fourth modified embodiment of the embryo transfer device in the first embodiment.
{Fig. 13}
   Fig. 13 is a plan view showing a fifth modified embodiment of the embryo transfer device in the first embodiment.
{Fig. 14}
   Fig. 14 is a plan view showing a sixth modified embodiment of the embryo transfer device in the first embodiment, as well as an example of the perimeter.
{Fig. 15}
   Fig. 15 is a plan view showing an example of the perimeter of the embryo transfer device in the first embodiment.
{Fig. 16}
   Fig. 16 is a plan view showing an example of the perimeter of the embryo transfer device in the first embodiment.
{Fig. 17}
   Fig. 17 is a plan view of an embryo transfer device of a second embodiment.
{Fig. 18}
   Fig. 18 is a side view showing a first other example embryo transfer device.
{Fig. 19}
   Fig. 19 is a plan view showing the first other example embryo transfer device.
{Fig. 20}
   Fig. 20 is a side view showing a second other example embryo transfer device.
{Fig. 21}
   Fig. 21 is a plan view showing a accommodated state of the second other example embryo transfer device.
{Fig. 22}
   Fig. 22 is a plan view showing the second other example embryo transfer device.
{Fig. 23}
   Fig. 23 is a plan view in a state in which the first other example embryo transfer device is used.

### {Description of Embodiments}

Now, an embryo transfer device 1 according to a first embodiment will be described below with reference to the drawings.

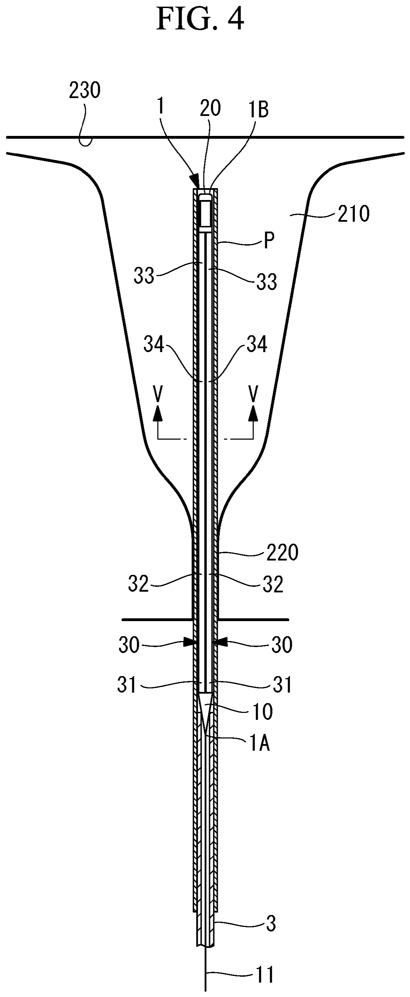

The embryo transfer device 1 is inserted into a uterine cavity 210 by using a tube P that is inserted into the uterine cervix (Fig. 4, etc.). In this embodiment, the embryo transfer device 1 is retained in the uterine cavity 210 for at least 48 hours. There are cases where the indwelling is performed for other lengths of time, such as at least 12 hours, at least 24 hours, or at least 72 hours.

The embryo transfer device 1 is used to cause an embryo to implant by retaining the embryo in the uterine cavity 210. In this embodiment, the term 'embryos' includes fertilized ova, fertilized oocytes, and blastocysts.

The embryo transfer device 1 in this embodiment is directed to maintain a state in which an embryo can come into contact with or in contact with the endometrium of a uterus, such as the uterine cavity 210, thereby improving the probability of implantation of the embryo. States that occur for the purpose of implantation as the state in contact mentioned above include a state in which the embryo is attached to the endometrium and a state in which the embryo penetrates into the endometrium. In one example, the embryo transfer device 1 helps the embryo enter the state in which the embryo is attached to the endometrium.

The embryo transfer device 1 should preferably be configured so as to avoid exerting any unnecessary force or influence on the uterus as much as possible. For example, it is expected that a reduction in the force applied to the endometrium from the embryo transfer device 1 disposed in the uterine cavity 210 contributes to successful implantation. Obviously, it is also important that damage to the uterine cervix 220, the uterine cavity 210, the body of the patient, the mind of the patient, etc. at the time of insertion/withdrawal of the embryo transfer device 1 is small. Meanwhile, in a situation where motion of intracorporeal organs including the uterus, motion of the body accommodating the uterus, and the like arise, it is necessary to reliably retain the embryo transfer device 1 in the uterine cavity 210, thereby maintaining a state in which the embryo can come into contact with or is in contact with the endometrium for the purpose of implantation. Regarding these conditions, there is a tendency that improving one condition results in deterioration of another condition. For example, a contraceptive device is something that is to be reliably retained in the uterine cavity 210; meanwhile, the adoption of this configuration leads to the application of relatively large force at various positions within the uterine cavity 210, high possibility of the application of similar force to the vicinity of the endometrium with which the embryo should come into contact, etc., which are likely to reduce the success rate of implantation.

The configuration of the embryo transfer device 1 in this embodiment, shown in Figs. 1 to 8, will be described below.

The embryo transfer device 1 has a proximal end 1A and a distal end 1B and is inserted into the uterine cavity 210 from the distal end 1B side by using the tube P that is inserted from the orifice of the uterus. In this embodiment, the proximal end 1A and the distal end 1B are the proximal end and the distal end of the embryo transfer device 1 when the embryo transfer device 1 is disposed in the tube P, as shown in Fig. 4. The embryo transfer device 1 includes: a proximal end portion 10 that forms at least a portion of the proximal end 1A; and an embryo accommodation portion 20 that is provided on the distal end 1B side and that accommodates an embryo. In the description of this embodiment, as shown in Figs. 1 to 3, the X axis passes through the proximal end portion 10 and the embryo accommodation portion 20, the Y axis, which is orthogonal to the X axis, extends in the direction in which expandable frame parts 30 are expanded, which will be described later, and the Z axis is orthogonal to the X axis and the Y axis. Note that although the distal end 1B is at the end of the embryo accommodation portion 20 in this embodiment, there may be cases where the distal end 1B is at another portion of the embryo transfer device 1.

The embryo transfer device 1 in this embodiment includes two expandable frame parts 30 that connect the proximal end portion 10 and the embryo accommodation portion 20. One end of each of the expandable frame parts 30 is connected to the proximal end portion 10, and the other end thereof is connected to the embryo accommodation portion 20. Each of the expandable frame parts 30 is formed of a material having rubber elasticity. Examples of the material having rubber elasticity include rubber materials such as silicone rubber, as well as other elastic materials.

Other kinds of rubber materials can be used as the rubber material. The rubber hardness of the rubber material in this embodiment falls within a range not exceeding 90 points when measured with a type-A durometer under the condition of 23 ± 2 °C; however, there is no limitation thereto. For example, the rubber hardness of the rubber material in this embodiment falls within a range of 10 to 90 points when measured with a type-A ASKER rubber hardness tester manufactured by KOBUNSHI KEIKI CO., LTD. under the condition of 23 ± 2 °C. The values of 10 to 90 points indicate durometer hardness in JIS K 6253. Since each of the expandable frame parts 30, etc. in this embodiment is thin, approximate values of a type-A durometer (type-A ASKER rubber hardness tester) may be measured with a durometer having the product name GS-680sel and manufactured by TECLOCK, and the rubber hardness of each of the expandable frame parts 30 is identified by using the approximate values. Instead of GS-680-sel, a micro rubber hardness tester having the product name MD-1 capa and manufactured by KOBUNSHI KEIKI CO., LTD. may be used.

Note that, in this embodiment, assuming that the Young's modulus of a contracted muscular layer located under the endometrium is around 10 MPa, the rubber hardness is set as described above so that the Young's modulus of the material of each of the expandable frame parts 30 will be equivalent thereto or less. This setting may afford the advantage that each of the expandable frame parts 30 is not recognized as a foreign object in the uterine cavity 210. Note that the Young's modulus of blood vessels under the endometrium may be lower than the Young's modulus mentioned above. In consideration of the Young's modulus of the blood vessels, etc., there are cases where the Young's modulus of the material of each of the expandable frame parts 30 should be even lower. In such cases, the rubber hardness of the material of each of the expandable frame parts 30 is set to be not higher than 60 points or not higher than 50 points.

Note that when it is difficult to measure the rubber hardness of each of the expandable frame parts 30, etc. with a type-A ASKER rubber hardness tester, it is possible to form a sheet of the same material having a thickness of 6 to 10 mm by using a mold, to measure the hardness of the sheet with a type-A ASKER rubber hardness tester under the condition of 23 ± 2 °C, and to use the measurement result as the rubber hardness of each of the expandable frame parts 30.

Preferably, the proximal end portion 10 and the embryo accommodation portion 20 are also formed of a material having a Young's modulus (rubber hardness) falling in the same range. In this embodiment, the proximal end portion 10, the embryo accommodation portion 20, and the two expandable frame parts 30 are integrally formed by using a mold, and the proximal end portion 10, the embryo accommodation portion 20, and the two expandable frame parts 30 are formed of the same material. The structure of the embryo transfer device 1 is not limited to this example. In each of the embodiments, there are cases where materials having rubber elasticity are simply referred to as elastic materials.

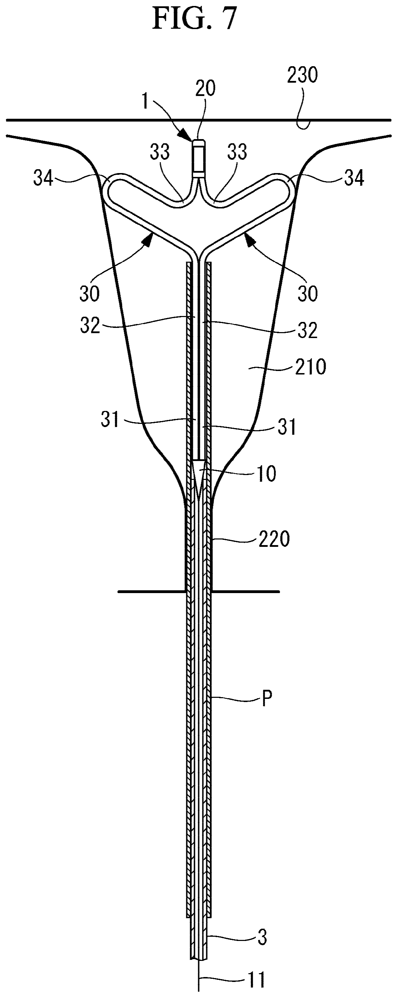

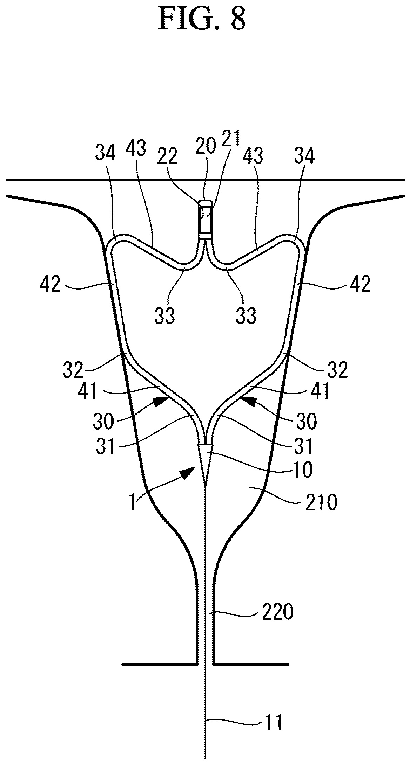

When accommodated, i.e., when disposed in the tube P for the purpose of insertion into the uterine cavity 210, the two expandable frame parts 30 become a state of being close to each other due to elastic deformation (Fig. 4). At this time, in this embodiment, portions of the two expandable frame parts 30 come into contact with each other. When released from the tube P, the two expandable frame parts 30 are expanded in the direction along the Y axis due to the restoring force of the elastic deformation (Figs. 7 and 8). Hereinafter, there are cases where the "direction along the X axis", the "direction along the Y axis", and the "direction along the Z axis" are referred to as the "X direction", the "Y direction, and the "Z direction", respectively.

In this embodiment, when released from the tube P, the two expandable frame parts 30 move due to the restoring force of the elastic deformation so that at least intermediate sections thereof in the length direction go away from each other and go away from the X axis, and are expanded due to this movement. Note that the two expandable frame parts 30 are allowed to also move in the direction along the Z axis, etc. at the time of the expansion. That is, expansion in the Y direction refers to the two expandable frame parts 30 moving as described above at least in the Y direction. Note that the movement of the two expandable frame parts 30 in the Y direction at the time of expansion should preferably be larger than the movements in the X direction and in the Z direction. In this case, it can be said that the two expandable frame parts 30 move mainly in the Y direction at the time of expansion.

Here, a typical example of the tube P is a tube made of a plastic material. In order to reduce the damage to the uterus at the time of insertion/withdrawal, which is mentioned earlier, the outer diameter of the tube P should preferably be not greater than 2.5 mm, more preferably not greater than 2.0 mm, and further preferably not greater than 1.5 mm. Accordingly, the inner diameter of the tube B becomes not greater than 2.3 mm, not greater than 1.8 mm, not greater than 1.3 mm, etc. There are cases where the tube P is called a guide, a sheath, or the like and there are also cases where the tube P is called a catheter, and it is also possible to use tubes having other names.

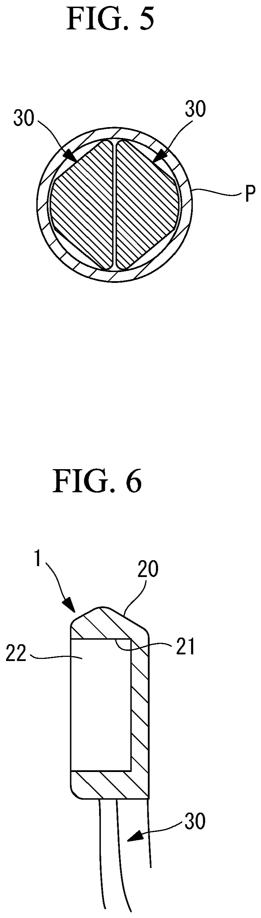

In order to dispose the embryo transfer device 1 in the hollow section of the tube P, which is thin as described above, each of the expandable frame parts 30 is formed to have a small cross-sectional area. For example, in the case where the inner diameter of the tube P (the diameter of the interior of the hollow section) is 1.8 mm, each of the two expandable frame parts 30 is to be disposed in a narrow area corresponding to one half of the cross-sectional area of the hollow section of the tube P. For example, as shown in Fig. 5, each of the expandable frame parts 30 may have a sectional shape forming an isosceles triangle, a shape similar to an isosceles triangle, or the like. The sectional shape of each of the expandable frame parts 30 may also be a semicircular shape, a shape in between these shapes, or a shape similar to one of these shapes. This configuration makes it possible to maximize the cross-sectional area of each of the expandable frame parts 30 in the narrow area mentioned above. This contributes to disposing the embryo accommodation portion 20 at a desired site which will be described later.

The cross-sectional area of the proximal end portion 10 suffices to be equivalent to or less than that of the hollow section of the tube P. In this embodiment, the proximal end portion 10 has a cylindrical or conical shape having a maximum diameter that is about 0.1 mm less than the inner diameter of the tube P. The proximal end portion 10 may have other shapes. In this embodiment, an end of a string 11 for withdrawal is fixed to the proximal end portion 10. In this embodiment, it can also be said that the proximal end portion 10 is a part that is pushed with a push rod 3. In another embodiment, the proximal end portion 10 is the place from which the string 11 comes out from the embryo transfer device 1. In yet other embodiments, there are cases where the string 11 does not come out from the proximal end portion 10, and there are also cases where the proximal end portion 10 is not pushed with the push rod 3.

The embryo accommodation portion 20 is opened in the direction along the Z axis. In this embodiment, as shown in Figs. 1, 6, and etc., the embryo accommodation portion 20 has a recess 21 recessed in the direction along the Z axis, and the opening of the recess 21 is opened in the direction along the Z axis. An embryo is accommodated in the recess 21. In this embodiment, the direction of opening of the opening 22 is the direction precisely along the Z axis. However, as long as the angle made by the direction of opening and the Z axis is not greater than 60°, preferably not greater than 45°, or more preferably not greater than 30°, those angles leads to attaining the advantage that will be described later, and it can be said that the opening 22 is opened in the direction along the Z axis. In this embodiment, the direction of opening is the direction orthogonal to the virtual plane defined by the edge of the opening 22. Even if the angle made by the direction of opening and the Z axis exceeds 60°, there are cases where the advantage that will be described later can be expected depending on the shape of the uterus, etc.

Since the embryo accommodation portion 20 is opened in the direction along the Z axis, as described above, when the embryo transfer device 1 is disposed in the uterine cavity 210, the state in which the embryo in the embryo accommodation portion 20 can come into contact with or is in contact with the endometrium is maintained.

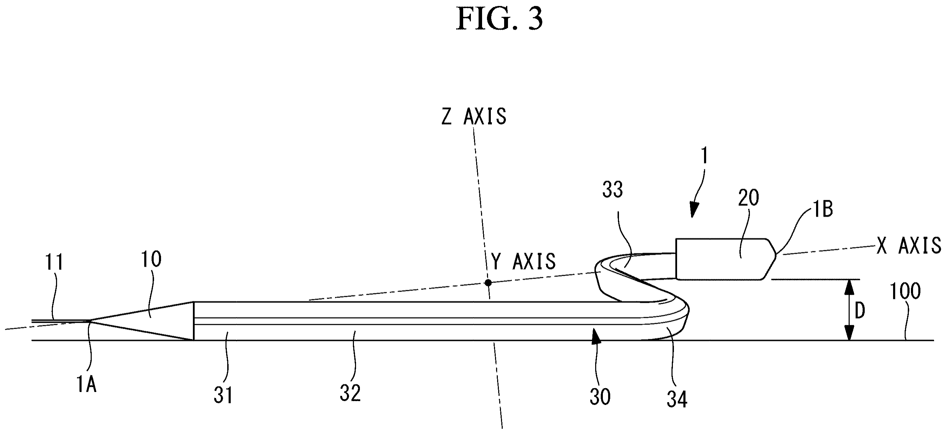

The shape of each of the expandable frame parts 30 in a no-load state, in which no load is applied to each of the expandable frame parts 30, will be described below. The no-load state refers to, for example, a state in which the embryo transfer device 1 is placed on a horizontal surface 100, as shown in Fig. 3, whereby only gravity is applied to the embryo transfer device 1. In Fig. 3, one end side and the intermediate section of each of the expandable frame parts 30 is in contact with the horizontal surface 100.

In the no-load state, each of the expandable frame parts 30 includes: a first bent section 31 that is curved in the direction away from the X axis from the one end toward the other end; and a second bent section 32 that is disposed at the other end side with respect to the first bent section 31 and that is bent in the direction going nearer to the X axis from the one end toward the other end.

Furthermore, in the no-load state, each of the expandable frame parts 30 includes: a third bent section 33 that is disposed at the other end side with respect to the second bent section 32 and that is bent in the direction away from the X axis from the other end toward the one end. Furthermore, in the no-load state, each of the expandable frame parts 30 includes a fourth bent section 34 that is disposed at the other end side with respect to the second bent section 32 and is disposed at the one end side with respect to the third bent section 33, and that is bent in the direction going nearer to the X axis from the one end toward the other end.

In each embodiment, the section between the first bent section 31 and the second bent section 32 is referred to as a first connecting section 41, the section between the second bent section 32 and the fourth bent section 34 is referred to as a second connecting section 42, and the section between the fourth bent section 34 and the third bent section 33 is referred to as a third connecting section 43.

As shown in Figs. 4 and 7, when the embryo transfer device 1 is disposed in the tube P, there are cases where the curvature (degree of curving) of each of the bent sections 31, 32, 33, and 34 becomes relaxed to be almost linear. When the embryo transfer device 1 is disposed in the tube P, the first bent section 31, the second bent section 32, the fourth bent section 34, the third bent section 33, and the embryo accommodation portion 20 are disposed in this order from the proximal end 1A side toward the distal end 1B side. Note that there are cases where the entire embryo transfer device 1 is accommodated in the tube P, and there may also be cases where a portion of the embryo transfer device 1, such as a portion of the proximal end portion 10 or the embryo accommodation portion 20, is exposed from the tube P.

For example, when disposing the embryo transfer device 1 disposed in the tube P into the uterine cavity 210, first, a user inserts one end side of the tube P into the uterine cervix 220 from the external orifice of the uterus, as shown in Fig. 4. Note that the user is, for example, a practitioner, and the embryo accommodation portion 20 is disposed at the distal end side of the tube P, while the proximal end portion 10 is disposed at the proximal end side of the tube P (Fig. 4, etc.). Then, for example, while applying a force directed toward the distal end of the tube P to the push rod 3 inserted into the tube P from the proximal end thereof, the user moves the tube P in the direction of withdrawal from the uterine cervix 220. In this embodiment, the push rod 3 has a thin pipe shape formed of a plastic, a metal, or the like. However, there are cases where the push rod 3 is formed of other materials and has other shapes.

Accordingly, as shown in Fig. 7, the embryo accommodation portion 20 and the third bent section 33 are first released from the tube P in the uterine cavity 210. Then, the fourth bent section 34, the second bent section 32, the first bent section 31, and the proximal end portion 10 are released from the tube P in this order, whereby the embryo transfer device 1 is disposed in the uterine cavity 210 (Fig. 8).

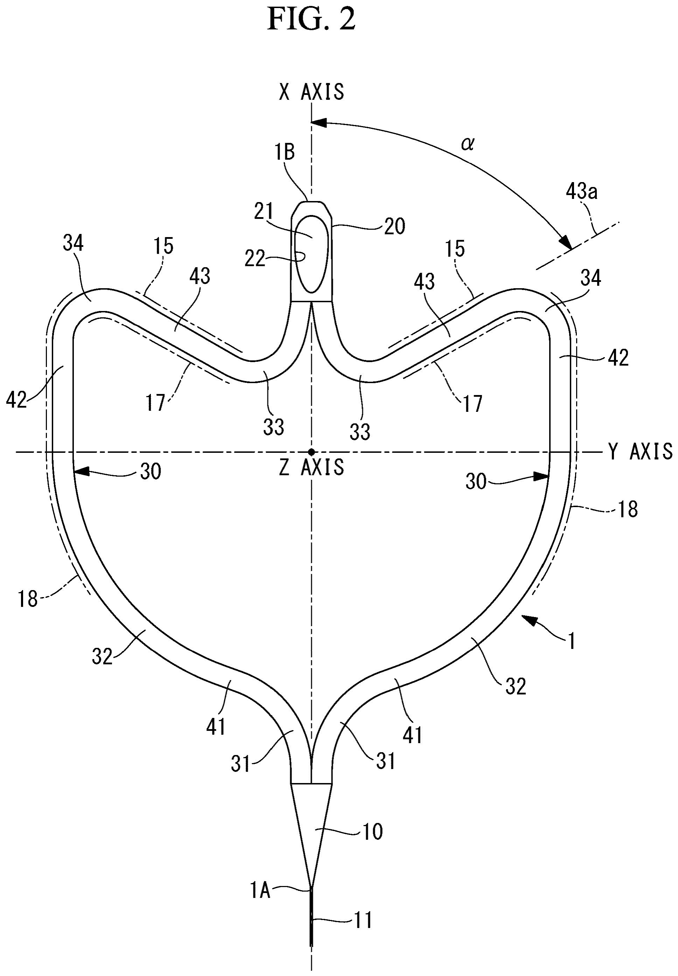

By the time when the one ends of the expandable frame parts 30 become released from the tube P after the other ends thereof become released, the two expandable frame parts 30 are expanded in the Y direction. As a result of this expansion, the third connecting section 43 of each of the expandable frame parts 30 is disposed so as to extend mainly in the Y direction when viewed from the direction along the Z axis. In one example, an extending direction 43a (Fig. 2), which is the direction of extension of the third connecting section 43, is the direction in which the straight line interconnecting both ends of the third connecting section 43 extends. When the angle made by the extending direction 43a and the Y axis when viewed from the direction along the Z axis is less than 45°, it can be said that the third connecting section 43 extends mainly in the Y direction.

In Fig. 2, the third connecting section 43 extends linearly when viewed from the direction along the Z axis. However, the third connecting section 43 may be curved when viewed from the Z axis direction. Even in this case, it is possible to identify the direction that the abovementioned straight line interconnecting both ends thereof extends. Furthermore, there are cases where the third bent section 33 and the fourth bent section 34 are directly connected to each other, and there are cases where the third bent section 33 and the fourth bent section 34 are connected to each other via a short third connecting section 43. In these cases, in each of the expandable frame parts 30, a prescribed range including the connecting section between the third bent section 33 and the fourth bent section 34 functions as the third connecting section 43. The prescribed range lies, for example, in a range extending from the other end toward the one end of each of the expandable frame parts 30 obliquely toward the deep side of the uterine cavity 210. The deep side refers to the distal end 1B side in the direction along the X axis.

While other portions of the embryo transfer device 1 come into contact with the uterus, in this embodiment, widthwise outer sections 18 (Fig. 2) of the two expandable frame parts 30, constituting both sides of the embryo transfer device 1 in the Y direction, come into contact with the inner walls of the uterine cavity 210. As shown in Fig. 8, the inner walls are typically the walls of the uterine cavity 210 on both sides in the width direction of the body. There are cases where the width direction is referred to as the width direction of the uterine cavity 210. The contact serves as resistance for movement of the widthwise outer sections 18 in relation to the uterus. Note that the one end side of each of the expandable frame parts 30 with respect to the widthwise outer section 18 also works as a movement resistance. Note that, in this embodiment, as shown in Fig. 2, a portion of the second bent section 32, a portion of the fourth bent section 42, and the second connecting section 42 constitute the widthwise outer section 18, or a portion of the fourth bent section 34 and the second connecting section 42 constitute the widthwise outer section 18. In either case, in this embodiment, the widthwise outer sections 18 constituting intermediate sections of the individual expandable frame parts 30 come into contact with the walls on both sides in the width direction of the uterine cavity 210.

As described above, the widthwise outer sections 18 are disposed at positions more distant from the X axis than the embryo accommodation portion 20. Thus, an advantage is afforded in that the embryo accommodation portion 20 is hardly disposed on the outer sides in the width direction of the uterine cavity 210. Furthermore, with this configuration, an advantage is afforded in that the embryo is prevented or inhibited from being disposed on the oviduct side.

Each of the expandable frame parts 30 is expanded in the Y direction with the one end thereof connected to the proximal end portion 10 and the other end thereof connected to the embryo accommodation portion 20. Thus, with each of the expandable frame parts 30, which is thin and is formed of a material having rubber elasticity as described earlier, compared with the case where the embryo accommodation portion 20 is supported with a thin rubber rod that extends from the proximal end portion 10 along the X axis and that can be inserted into the tube P, an advantage is afforded in that the position of the embryo accommodation portion 20 in the Y direction tends to be stable.

Furthermore, the embryo accommodation portion 20 becomes supported via the part of third connecting sections 43 in relation to the pair of widthwise outer sections 18, and each of the third connecting sections 43 extends mainly in the Y direction. Accordingly, the advantage that the position of the embryo accommodation portion 20 in the Y direction tends to be stable is attained more reliably.

Note that, in this embodiment, each of the expandable frame parts 30 is expanded in the Y direction with the one end thereof connected to the proximal end portion 10 and the other end thereof connected to the embryo accommodation portion 20. With this configuration, the intermediate sections of the pair of expandable frame parts 30 are disposed on both side in the width direction of the uterine cavity 210, with the other end sides of the pair of expandable frame parts 30 disposed on the deep side of the uterine cavity 210, and with the one end sides of the pair of expandable frame parts 30 disposed on the entry (orifice) side of the uterine cavity 210. In this embodiment, there are cases where both sides in the width direction of the uterine cavity 210 are referred to as both side walls.

As described above, the pair of expandable frame parts 30 are disposed so as to span the both side walls, the deep side, and the entry side in the uterine cavity 210. In one example, the intermediate sections (widthwise outer sections 18) of the pair of expandable frame parts 30 are disposed along the both side walls in the uterine cavity 210, with the one end sides of the pair of expandable frame parts 30 disposed between the intermediate sections. Furthermore, the other end sides of the pair of expandable frame parts 30 are disposed at the deeper side of the uterine cavity 210 than the one end sides thereof and are disposed between the intermediate sections. In this embodiment, there are cases where such an arrangement of the pair of expandable frame parts 30 is referred to as a ring-shaped arrangement.

Note that, in the case of this embodiment, to be more specific, the pair of expandable frame parts 30, the embryo accommodation portion 20, and the proximal end portion 10 are disposed so as to span the both side walls, the deep side, and the entry side in the uterine cavity 210; however, even the range of the pair of expandable frame parts 30 alone can be referred to as the ring-shaped arrangement mentioned above. Preferably, the pair of expandable frame parts 30 should occupy a length corresponding to at least 70%, preferably at least 80%, or more preferably at least 90% in the circumferential direction of the ring shape in order to realize the stabilization of the position of the embryo accommodation portion 20 in the uterine cavity 210 and a reduction in the force applied from the embryo transfer device 1 to the endometrium.

With each of the expandable frame parts 30, which is thin and is formed of a material having rubber elasticity as described earlier, the ring-shaped arrangement mentioned above affords the advantage that the position of the embryo accommodation portion 20 tends to be stable. That is, when the embryo accommodation portion 20 is moved in the Y direction, in one example, for example, a force around the center of the ring shape is applied to the entirety of each of the expandable frame parts 30. With each of the expandable frame parts 30, a movement resistance occurs due to contact with the uterine cavity 210 or the like. Thus, each of the expandable frame parts 30, which is thin and has rubber elasticity, affords the advantage that the ring-shaped arrangement mentioned above serves to stabilize the position of the embryo accommodation portion 20. This advantage of the ring-shaped arrangement can be attained even if the angle made by the extending direction 43a and the Y axis when viewed from the direction along the Z axis is not less than 45°.

Furthermore, in this embodiment, the extending direction 43a is inclined toward the deep side of the uterine cavity 210 from the other end toward the one end of each of the expandable frame parts 30, and the distal end 1B side of the pair of expandable frame parts 30 has a square shoulder shape. Alternatively, the arrangement may be such that the extending direction 43a is inclined toward the entry side (the proximal end 1A side in the direction along the X axis) of the uterine cavity 210 from the other end toward the one end of each of the expandable frame parts 30 and such that the distal end 1B side of the pair of expandable frame parts 30 has a sloping shoulder shape. The advantage of the ring-shaped arrangement described above can be afforded even in the case of a sloping shoulder shape.

Furthermore, with the ring-shaped arrangement mentioned above, the advantage of stabilizing the position of the embryo accommodation portion 20 in the X direction can also be expected. In order to stabilize the position of the embryo accommodation portion 20 mainly in the X direction, it is also conceivable to make the angle made by the extending direction 43a and the Y axis when viewed from the direction along the Z axis not less than 45°.

In order to realize a reduction in the force applied from the embryo transfer device 1 to the endometrium while stabilizing the position of the embryo accommodation portion 20 in the uterine cavity 210, the length of each of the expandable frame parts 30 should be preferably not less than 25 mm, more preferably not less than 30 mm, and further preferably not less than 40 mm.

Furthermore, in order to stabilize the position of the embryo transfer device 1 and the embryo accommodation portion 20 in the uterine cavity 210, the dimension of the embryo transfer device 1 along the Y direction in the no-load state should be preferably not less than 15 mm, more preferably not less than 20 mm, and further preferably not less than 30 mm. Note that, in this embodiment, both ends of the embryo transfer device 1 in the Y direction are the intermediate sections of the pair of expandable frame parts 30.

In consideration of differences among individuals regarding the shape, size, conditions, etc. of the uterine cavity 210, a plurality of kinds of embryo transfer devices 1 with different lengths of the expandable frame parts 30 may be prepared.

In this embodiment, the recess 21, in which the embryo is accommodated in the embryo accommodation portion 20, is disposed on the distal end 1B side with respect to the other end of each of the expandable frame parts 30. Since it is believed that the probability of implantation is higher on the deeper side of the uterine cavity 210, in the case of a patient matching the condition, the configuration described above affords the advantage of serving to improve the probability of implantation.

In this embodiment, in the no-load state, the angle α at the distal end 1B side (the deep side of the uterine cavity 210) of the angles made by the extending direction 43a and the X axis when viewed from the direction along the Z axis is less than 90° (Fig. 2). That is, in the no-load state, the other end side of each of the expandable frame parts 30 has a distal-direction inclined section 15 extending obliquely to the deep side (the distal end 1B side in the direction along the X axis) of the uterine cavity 210 from the other end side toward the one end side. The one end side of the third bent section 33 may also function as the distal-direction inclined section 15. Alternatively, in the no-load state, each of the expandable frame parts 30, on the one end side with respect to the third bent section 33, has a section (the third connecting section 43, a portion of the fourth bent section 34, etc.) disposed on the deeper side of the uterine cavity 210 than the terminal of the one end of the third bent section 33.

There are cases where this configuration affords the advantage of contributing to preventing the embryo from being disposed on an oviduct side. Furthermore, in this embodiment, when the pair of widthwise outer section 18 come closer to each other due to a force from the uterus, etc. compared with the no-load state, rather than a force in the direction approaching the fundus 230 of the uterus, a force in the direction away from the fundus 230 of the uterus tends to be applied to the embryo accommodation portion 20. This leads to the advantage of avoiding the application of unnecessary force to the uterus as much as possible.

In this embodiment, the other end side of the fourth bent section 34 and the third connecting section 43 constitute a Z-direction sloped section 17 sloped in the direction along the Z axis in the no-load state. The one end side of the third bent section 33 may also function as the Z-direction sloped section 17. That is, the Z-direction sloped section 17 exists in the range from the other end of each of the expandable frame parts 30 to the second bent section 32. Note that the Z-direction sloped section 17 suffices to exist at least in a portion of the range mentioned above. Note that the "range from the other end to the second bent section 32" refers to a range including the range in which the second bent section 32 exists.

The slope angle of the Z-angle sloped section 17 with respect to the horizontal surface 100 is preferably not less than 2°, more preferably not less than 3°, and further preferably not less than 4°. Furthermore, the slope angle mentioned above is preferably not greater than 80°, more preferably not greater than 60°, and further preferably not greater than 45° or 30°. The Z-direction sloped section 17 offsets the embryo accommodation portion 20 in the Z direction with respect to the widthwise outer section 18. The amount of offset corresponds to the portion indicated as a distance D in Fig. 3. The amount of the offset is preferably not less than 1.5 mm, more preferably not less than 2.5 mm, and further preferably not less than 3.5 mm.

With this configuration, an advantage is afforded in that even in the case where intracorporeal organs such as the uterus move or the body accommodating the uterus moves, the state in which the opening 22 of the embryo accommodation portion 20 is in contact with or is in proximity to the endometrium tends to be maintained. Even in the case where the amount of offset mentioned above is less than 1.5 mm or in the case where the Z-direction sloped section 17 is not provided, there are cases where an advantage similar to the above advantage is attained depending on other conditions. That is, an embryo transfer device 1 not including the Z-direction sloped section 17 can attain an advantage equivalent to the advantage of the embryo transfer device 1 including the Z-direction sloped section 17, and the embryo transfer device 1 not including the Z-direction sloped section 17 is also useful.

In the embryo transfer device 1 in this embodiment, the two expandable frame parts 30, as a result of the expansion thereof and with the ring-shaped arrangement thereof, sets the position of the embryo accommodation portion 20 at a center side in the width direction of the uterine cavity 210. With this configuration, an advantage is afforded in that the embryo accommodation portion 20 is hardly disposed outside the width direction of the uterine cavity 210, which results in the advantage of preventing or inhibiting the embryo from being disposed on the oviduct side.

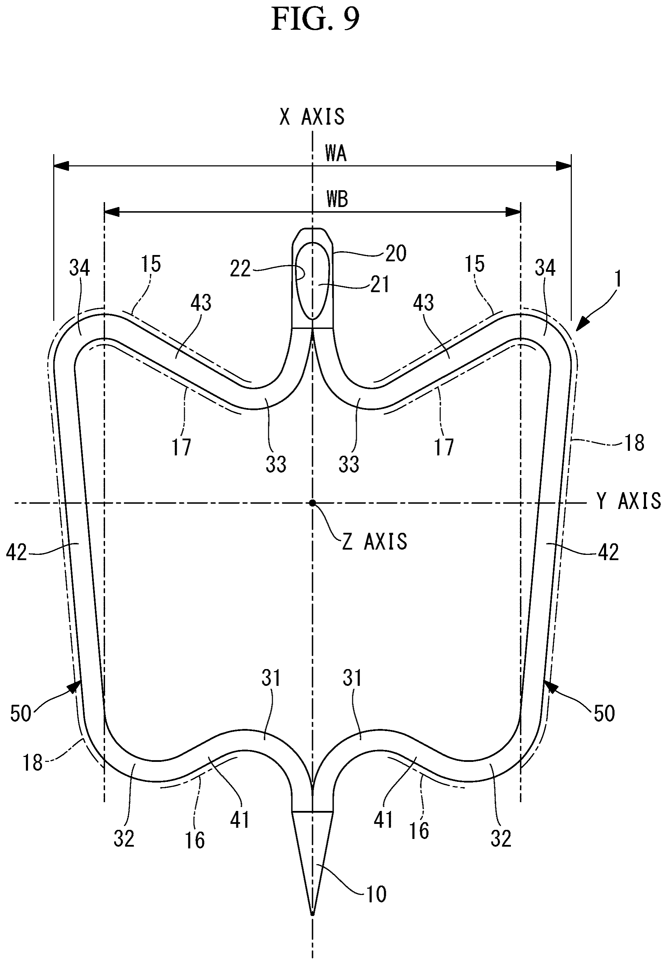

It is also possible to adopt expandable frame parts 50 shown in Fig. 9 instead of the expandable frame parts 30. Similarly to the expandable frame parts 30, each of the expandable frame parts 50 in the embodiment in Fig. 9 includes a first bent section 31, a second bent section 32, a third bent section 33, a fourth bent section 34, a first connecting section 41, a second connecting section 42, and a third connecting section 43.

Also in each of the expandable frame parts 50 in the embodiment in Fig. 9, a portion of the second bent section 32, a portion of the fourth bent section 34, and the second connecting section 42 constitute the widthwise outer section 18, or a portion of the fourth bent section 34 and the second connecting section 42 constitute the widthwise outer section 18.

In each of the expandable frame parts in the embodiment in Fig. 9, the first connecting section 41, which is the section extending from the terminal of the first bent section 31 on the other end side, or the first connecting section 41 and a portion of the second bent section 32 constitute a proximal-direction inclined section 16 extending obliquely toward the entry side (the proximal end 1A side in the direction along the X axis) of the uterine cavity 210 from the one end toward the other end.

Thus, in the embodiment in Fig. 9, an advantage is afforded in that even in the case where intracorporeal organs such as the uterus move or in the case where the body accommodating the uterus moves, the possibility of accidental discharging of the embryo transfer device 1 from the uterine cavity 210 is reduced.

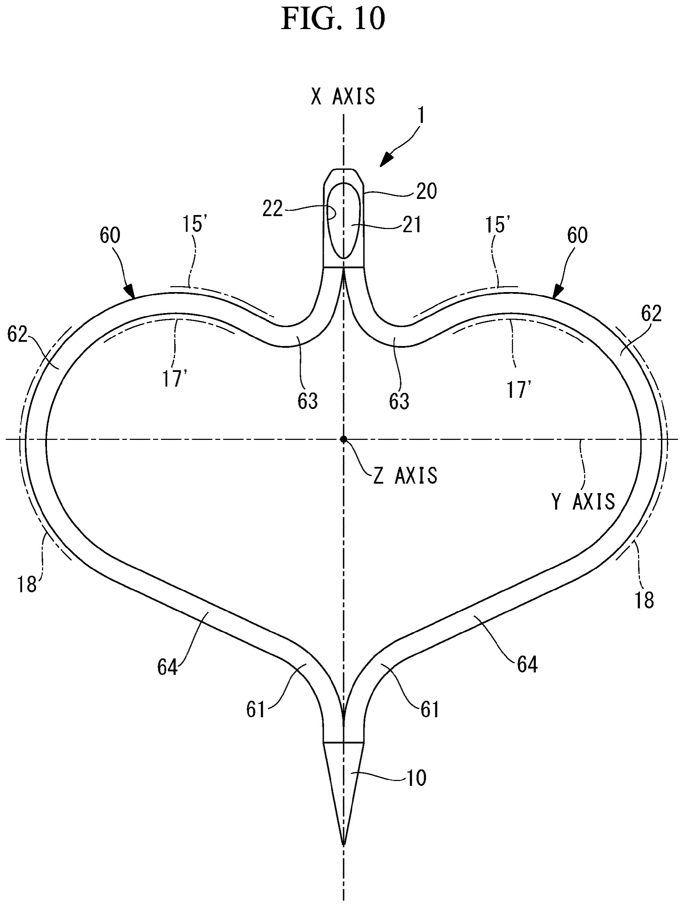

It is also possible to adopt expandable frame parts 60 shown in Fig. 10 instead of the expandable frame parts 30. In the no-load state, each of the expandable frame parts 60 includes: a first bent section 61 that is curved in the direction away from the X axis from the one end toward the other end; and a second bent section 62 that is disposed on the other end side with respect to the first bent section 61 and that is bent in the direction approaching the X axis from the one end toward the other end.

Furthermore, in the no-load state, each of the expandable frame parts 60 includes a third bent section 63 that is disposed on the other end side with respect to the second bent section 62 and that is bent in the direction away from the X axis from the other end toward the one end.

In the embodiment in Fig. 10, the section between the first bent section 61 and the second bent section 62 is referred to as a first connecting section 64. The section between the second bent section 62 and the third bent section 63 constitutes a second connecting section; however, in the case where the terminal of the second bent section 62 on the other end side is directly connected to the third bent section 63, as in the embodiment in Fig. 10, the second connecting section is substantially absent.

Also in the embodiment in Fig. 10, in the no-load state, the distal end side angle, which is located at a side of the distal end 1B, of the angles made by the other end side of the second bent section 62 and the X axis when viewed from the direction along the Z axis, the distal end side angle is less than 90°. That is, in the no-load state, a portion of the second bent section 62, which is the section extending from the terminal of the third bent section 63 on the one end side, constitutes a distal-direction inclined section 15' extending obliquely toward the deep side of the uterine cavity 210. The one end side of the third bent section 63 may also function as the distal-direction inclined section 15'.

Also in the embodiment in Fig. 10, the other end side of the second bent section 62 constitutes a Z-direction sloped section 17' sloped in the direction along the Z axis in the no-load state. The one end side of the third bent section 63 may also function as the Z-direction sloped section 17'. That is, the Z-direction sloped section 17' exists in the range from the other end of each of the expandable frame parts 60 to the second bent section 62. Note that the "range from the other end to the second bent section 62" refers to a range including the range in which the second bent section 62 exists.

In the embodiment in Fig. 10, the second bent section 62 may be configured such that the curvature thereof changes in the no-load state. Obviously, the curvature of each bent section may change in each embodiment. For example, the second bent section 62 may be configured such that the curvature thereof on the one end side is tight, the curvature thereof in the intermediate section thereof is loose, and the curvature thereof on the other end side is tight. By making the curvature on the one end side as described above, it is possible to provide a proximal-direction inclined section 16 as in the embodiment in Fig. 9.

Also in each of the expandable frame parts 60 in the embodiment in Fig. 10, the intermediate section of the second bent section 62, etc. constitute the widthwise outer section 18.

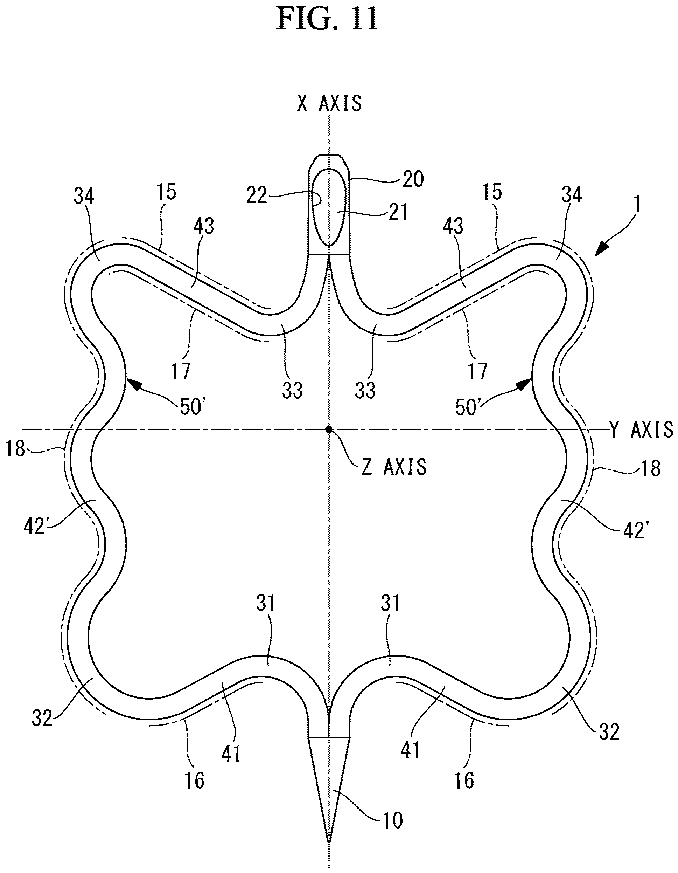

There may be cases where the expandable frame parts 50 shown in Fig. 9 have the shape shown in Fig. 11. In each of the expandable frame parts 50' in the embodiment in Fig. 11, the shape of the second connecting section is changed from that in the example shown in Fig. 9, and a second connecting section 42' in the embodiment in Fig. 11 is undulating when viewed from the direction along the Z axis.

Also in this case, the fourth bent section 34 is disposed on the other end side with respect to the second bent section 32, is disposed on the one end side with respect to the third bent section 33, and is bent in the direction approaching the X axis from the one end toward the other end. Furthermore, similarly to the embodiments in Figs. 1-3 and 9, etc., the other end side of the fourth bent section 34, the third connecting section 43, and the third bent section 33 of each of the expandable frame parts 50' function as a support section. A pair of support sections support the embryo accommodation portion 20 in relation to the pair of widthwise outer sections 18, and the embryo accommodation portion 20 is disposed on the inside in the width direction of the uterine cavity 210 with respect to the pair of widthwise outer sections 18. Thus, also with the embryo transfer device 1 shown in Fig. 11, an advantage similar to that of each of the embodiments described above can be afforded. In each embodiment, each bent section and each connecting section may be formed so as to undulate similarly.

In each of the embodiments described above, each support section extends mainly in the Y direction. It can be said that, for example, the range more distant from the X axis than a double dotted chain line shown in Fig. 9 when viewed from the direction along the Z axis or when imaged in the direction along the Z axis is the widthwise outer section 18. In the description of each embodiment, it can be said that when viewed from the direction along the Z axis can be considered as when imaged in the direction along the Z axis. The two double dotted chain lines extend in the direction along the X axis. In this embodiment, the distance WB between the two double dotted chain lines (the distance in the Y direction) is 8/10 of the width dimension WA, which is the dimension of the embryo transfer device 1 in the Y direction. In another example, WB is not less than 7/10 of WA, and WB may be not less than 9/10 of WA.

That is, in the pair of expandable frame parts 50, the widthwise outer sections 18 occupy the outer 3/10 range, the outer 2/10 range, or the outer 1/10 range of the dimension of the embryo transfer device 1 in the Y direction, constituting the portions on both outer sides of the embryo transfer device 1 in the Y direction. In each of the embodiments described above, the widthwise outer sections 18 can be set similarly. When viewed in another way, it can also be said that the widthwise outer sections 18 are sections that support the outer side ends in the Y direction of each support section extending mainly in the Y direction. The arrangement may be such that the portions on both outer sides of the embryo transfer device 1 in the Y direction constitute the widthwise outer sections 18, or the arrangement may be such that the outer 2/10 range of the dimension of the embryo transfer device 1 in the Y direction constitutes the widthwise outer sections 18.

In this embodiment, it is considered that each support section extends mainly in the Y direction if the angle made by the straight line passing through one end of the support section on the widthwise outer section 18 side and the other end of the support section (the other end of the expandable frame part 50) and the Y axis is less than 45°. That is, each support section extends in the Y direction rather than in the X direction.

Since each support section extends in the Y direction as described above, with each of the expandable frame parts 50, which is thin and is formed of a material having rubber elasticity as described earlier, compared with the case where the embryo accommodation portion 20 is supported with a thin rubber rod that extends from the proximal end portion 10 along the X axis and that can be inserted into the tube P, an advantage is afforded in that the position of the embryo accommodation portion 20 in the Y direction tends to be stable.

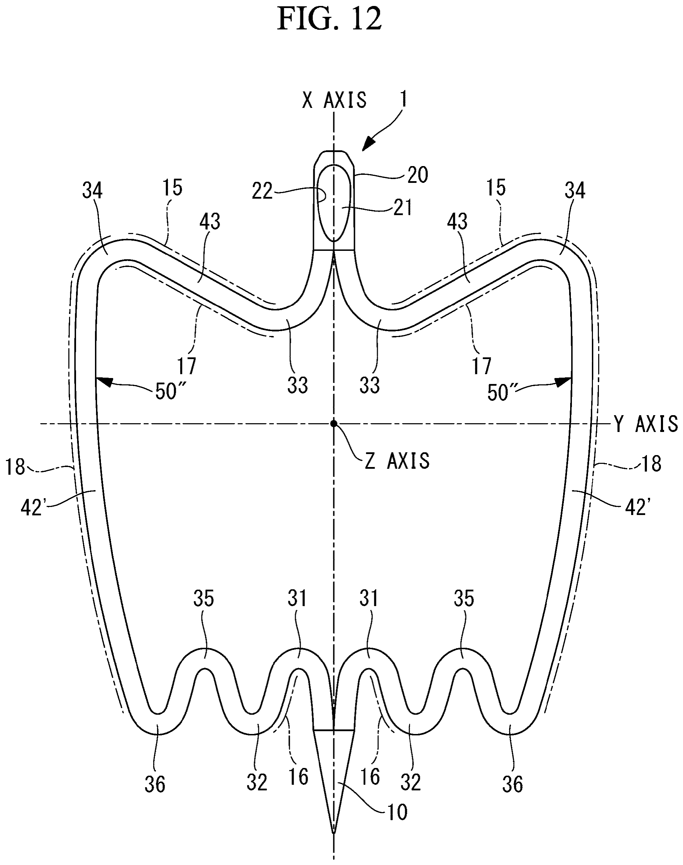

There may also be cases where the expandable frame parts 50 shown in Fig. 9 have the shape shown in Fig. 12. In each of expandable frame parts 50" in the embodiment in Fig. 12, compared with the example shown in Fig. 9, the shape of the second bent section 32 is changed, the intermediate section of a second connecting section 42' is arranged to have a curved shape convex toward the widthwise outer side, and a fifth bent section 35 and a sixth bent section 36 are added in the vicinity of the second bent section 32.

When the curvatures of the first bent section 31 and the second bent section 32 are made tighter and the fifth bent section 34 and the sixth bent section 36 similarly having tight curvatures are added, as described above, the volume of the expandable frame parts 50" in the vicinity of the proximal end portion 10 increases. With the increased volume section provided in the vicinity of the proximal end portion 10 as described above, similarly to the proximal-direction inclined section 16, an advantage is afforded in that the possibility of accidental discharge of the embryo transfer device 1 from the uterine cavity 210 is reduced. Note that the embodiment in Fig. 12 also includes the proximal-direction inclined section 16.

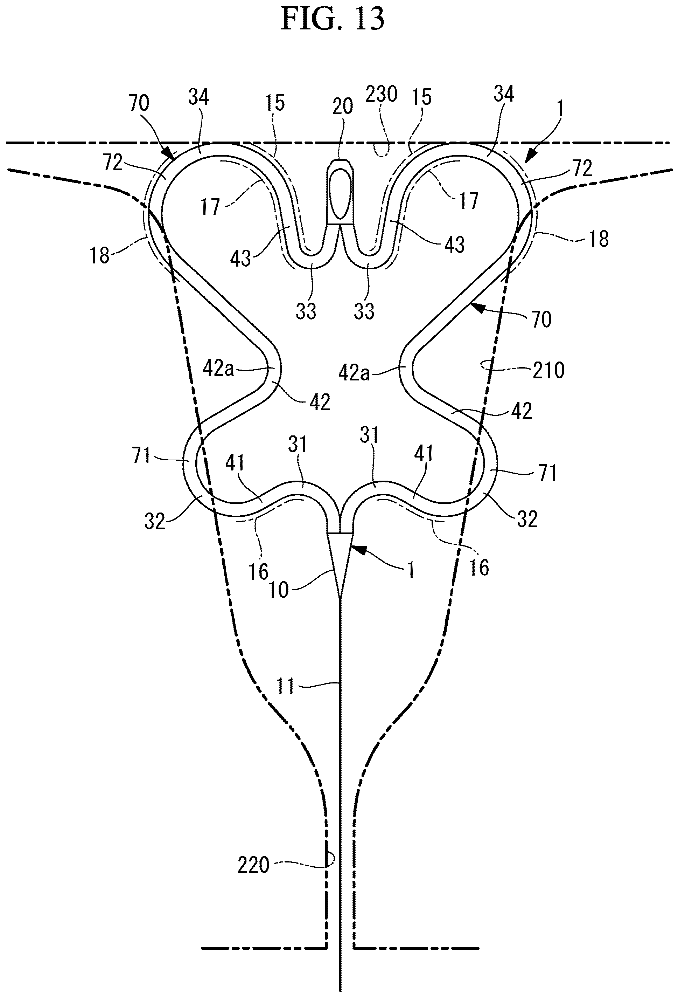

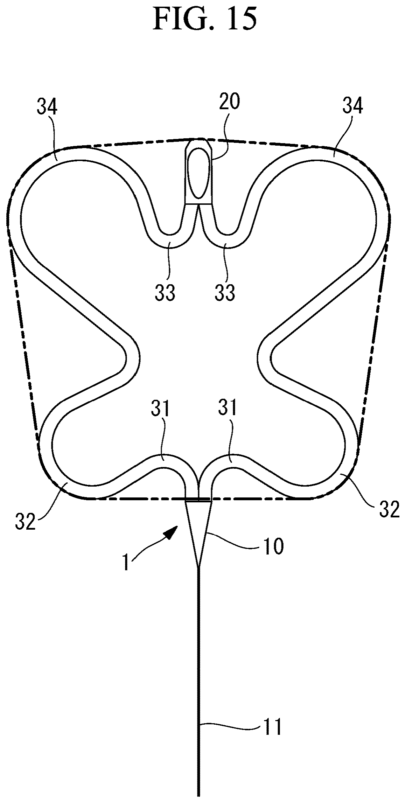

Note that, also in Figs. 9, 10, 11, and 12, the X axis passes through the proximal end portion and the embryo accommodation portion, which is also the case with Figs. 13 and 15, etc., which will be described later.

Discharge preventing sections for preventing the embryo transfer device 1 from being discharged from the uterine cavity 210, such as the increased volume section described above and the proximal-direction inclined section 16, are provided on the one end side of each of the expandable frame parts or in the proximal end portion 10.

When the embryo transfer device 1 is withdrawn from the uterine cavity 210 by pulling the string 11, in the increased volume section and the proximal-direction inclined section 16, each bent section is deformed such that the curvature thereof becomes loose, and thus the embryo transfer device 1 is withdrawn smoothly from the uterine cavity 210. With this configuration, an advantage is afforded in that it is possible to reduce damage to the uterine cervix 220, the uterine cavity 210, the body of the patient, the mind of the patient, etc. when the embryo transfer device 1 is withdrawn.

Furthermore, in each embodiment, the proximal end portion 10 has such a shape whose outer diameter gradually becomes smaller toward the proximal end 1A. Also with this configuration, an advantage is afforded in that it is possible to reduce damage to the uterine cervix 220, the uterine cavity 210, the body of the patient, the mind of the patient, etc. when the embryo transfer device 1 is withdrawn.

Furthermore, in each embodiment, the string 11 extends to the outside of the embryo transfer device 1 from the proximal end portion 10, and an end of the string 11 is disposed inside a portion formed of an elastic material, such as the proximal end portion 10. In this embodiment, the proximal end portion 10 is the portion where the string 11 comes out. Also with this configuration, an advantage is afforded in that it is possible to reduce damage to the uterine cervix 220, the uterine cavity 210, the body of the patient, the mind of the patient, etc. when the embryo transfer device 1 is withdrawn. Note that other methods may be used to connect the string 11 to a portion formed of an elastic material, such as the proximal end portion 10.

Note that, in the embodiments in Figs. 3 and 9, etc., it is also possible to adopt a second connecting section 42 that is bent so as to approach the X direction instead of the linear second connecting section 42. In this case, the second connecting section is bent in the direction away from the X axis from the one end toward the other end. In another example, the second connecting section 42 may be provided with a bent section that is bent in the direction away from the X axis from the one end toward the other end. These configurations are expected to contribute to the advantage of stabilizing the position of the embryo transfer device 1 in the uterine cavity 210 or the advantage of preventing accidental discharge of the embryo transfer device 1 from the uterine cavity 210.

It is also possible to adopt expandable frame parts 70 shown in Fig. 13 instead of the expandable frame parts 30. Similarly to the expandable frame parts 30, each of the expandable frame parts 70 in the embodiment in Fig. 13 includes a first bent section 31, a second bent section 32, a third bent section 33, a fourth bent section 34, a first connecting section 41, a second connecting section 42, and a third connecting section 43.

In each of the expandable frame parts 70 in the embodiment in Fig. 13, a portion of the fourth bent section 34 constitutes the widthwise outer section 18, or a portion of the fourth bent section 34 and a portion of the second bent section 32 constitute the widthwise outer section 18. Also in the embodiment in Fig. 13, similarly to the embodiment in Fig. 9, the distal-direction inclined section 15, the proximal-direction inclined section 16, and the Z-direction sloped section 17 are provided.

In the embodiment in Fig. 13, the second connecting section 42 is provided with a bent section 42a that is bent in the direction away from the X axis from the one end toward the other end. The entirety or a portion of the second connecting section 42 may have a shape that is convex in the direction approaching the X axis.

In the embodiment in Fig. 13, each of the expandable frame parts 70 includes: a first convex curved section 71 that is curved so as to have a shape convex in the direction away from the X axis; and a second convex curved section 72 that is disposed on the other end side with respect to the first convex curved section 71 and that is curved so as to have a shape convex in the direction away from the X axis.

With this configuration, in the embryo transfer device 1 in the embodiment in Fig. 13, the first convex curved section 71 and the second convex curved section 72 are disposed so as to positioned along both side walls of the uterine cavity 210 or come into contact with both side walls of the uterine cavity 210. In Fig. 13, an example of the schematic shape of the uterine cavity 210 is indicated with a double dotted chain line. Each of the expandable frame parts 70, when disposed in the uterine cavity 210, is deformed as appropriate depending on the shape of the uterine cavity 210. This configuration leads to the stabilization of the position of the embryo transfer device 1 in the uterine cavity 210, which makes it possible to attain the advantage that the position of the embryo accommodation portion 20 in the Y direction tends to be stable. It is considered that the degree of the advantage varies depending on the adjustment of the amounts of projection, the degrees of curving, etc. of the first convex curved section 71 and the second convex curved section 72.

Furthermore, in the embodiment in Fig. 13, the second convex curved section 72 has a shape that is also convex toward the deep side of the uterine cavity 210, and a portion of the second convex curved section 72 is disposed at the deeper side in the uterine cavity 210 than the embryo accommodation portion 20 and/or the distal end 1B. This may contribute to the prevention of contact of the embryo accommodation portion 20 and/or the distal end 1B with the fundus 230 of the uterus, a reduction in the force of the contact, etc. These leads to the advantage of avoiding the application of unnecessary force to the fundus 230 of the uterus as much as possible.

Note that, by using an inspection device such as an ultrasonic inspection device, it is possible to check, during insertion or after insertion, the status of each of the expandable frame parts in the uterine cavity 210 and the status of contact between each of the expandable frame parts and both side walls of the uterine cavity 210, the fundus 230 of the uterus, etc.

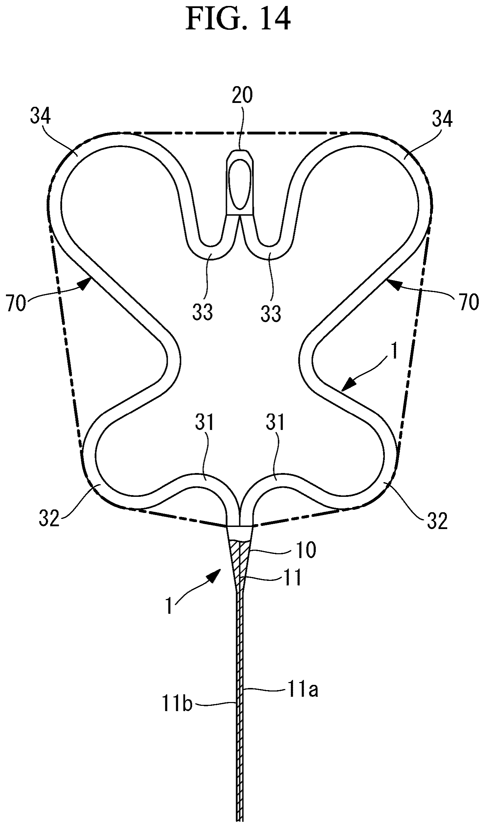

With the embryo transfer device 1 in the embodiment in Fig. 13, as indicated with a double dotted chain line in Fig. 14, it is also possible to define the periphery of the plurality of expandable frame parts 70 in the ring-shaped arrangement described earlier. The double dotted chain line in Fig. 14 is the shortest line drawn to encompass all the plurality of expandable frame parts 70 when viewed from the direction along the Z axis, which constitutes the perimeter of the ring-shaped part formed of the plurality of expandable frame parts 70.

It is also possible to define the perimeter of the plurality of expandable frame parts 70 as indicated with a double dotted chain line in Fig. 15. The embryo transfer device 1 in Fig. 15 is the type wherein the amount of projection of each of the second convex curved sections 72 in the X direction is small in the embodiment in Fig. 13. The double dotted chain line in Fig. 15 is the shortest line drawn to encompass the portion of the plurality of expandable frame parts 70 except the one end side thereof and the embryo accommodation portion 20 when viewed from the direction along the Z axis, which constitutes the periphery of the ring-shaped part formed of the plurality of expandable frame parts 70.

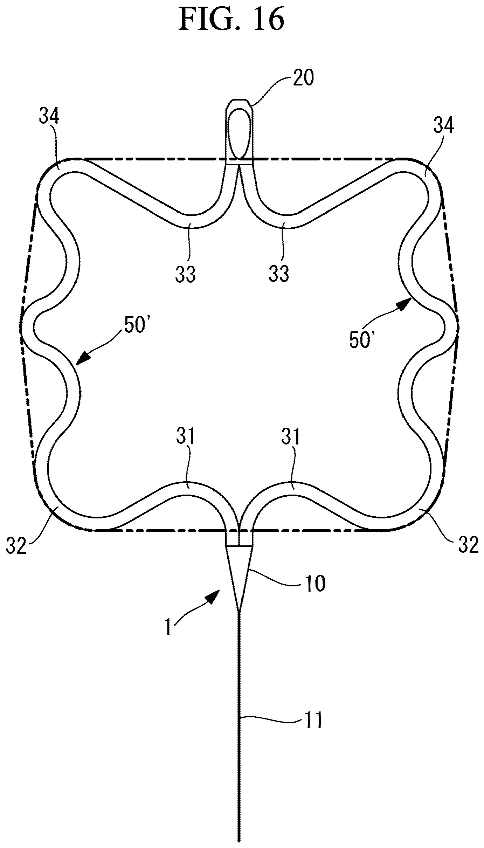

With the embryo transfer device 1 in the embodiment in Fig. 11, it is also possible to define the perimeter of the plurality of expandable frame parts 50' as indicated with a double dotted chain line in Fig. 16. The double dotted chain line in Fig. 16 is the shortest line drawn to encompass the portion of the plurality of expandable frame parts 50' except the one end side thereof when viewed from the direction along the Z axis, which constitutes the perimeter of the ring-shaped part formed of the plurality of expandable frame parts 70.

Also in the embodiments in Figs. 2, 10, 11, and 12, it is possible to define the perimeter of the ring-shaped part according to one of the above examples.

In order to realize a reduction in the force that is applied from the embryo transfer device 1 to the endometrium while stabilizing the position of the embryo accommodation portion 20 in the uterine cavity 210, the length of the perimeter of the ring-shaped part should be preferably not less than 60 mm, more preferably not less than 70 mm, and further preferably not less than 80 mm. Although not specifiable because of individual differences in the shape, size, conditions, etc. of the uterine cavity 210, the length of the perimeter of the ring-shaped part is not greater than 180 mm, not greater than 150 mm, or not greater than 125 mm.

In consideration of individual differences in the shape, size, conditions, etc. of the uterine cavity 210, a plurality of kinds of embryo transfer devices 1 with different lengths of the perimeter of the ring-shaped part may be prepared.

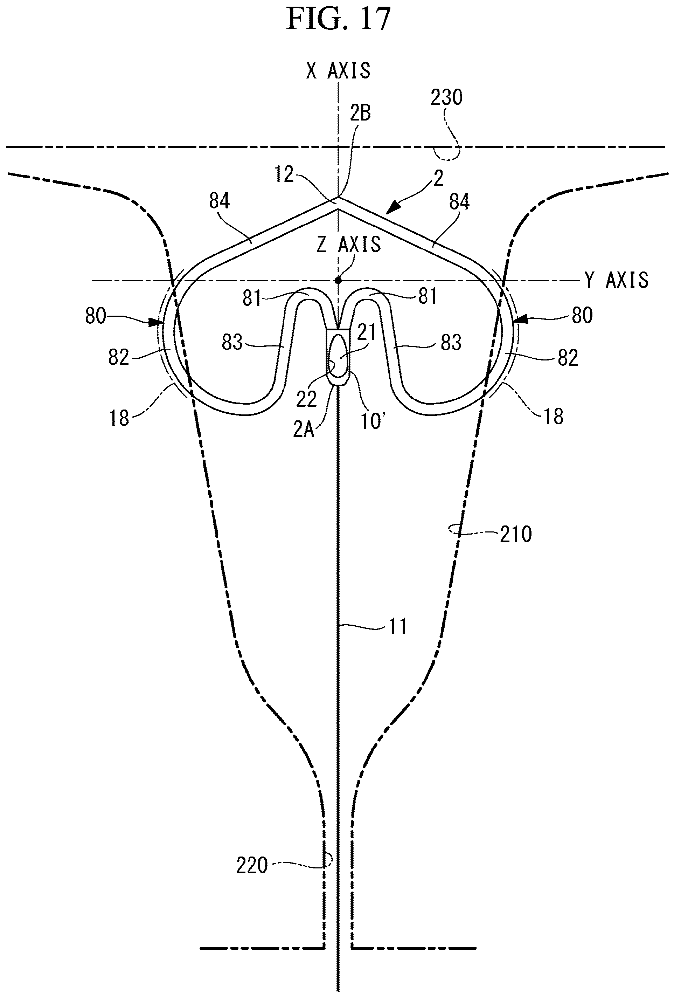

Now, an embryo transfer device 2 according to a second embodiment will be described below with reference to Fig. 17.

Similarly to the first embodiment, the embryo transfer device 2 in the second embodiment is inserted from a distal end 2B side into the uterine cavity 210 by using the tube P. The distal end 2B of the embryo transfer device 2 is a portion of a distal end portion 12. In a proximal end portion 10' forming a portion of a proximal end 2A of the embryo transfer device 2, the same recess 21 as that in the first embodiment is formed as an embryo accommodation portion. It can also be said that the proximal end portion 10' is an embryo accommodation portion. The embryo transfer device 2 in the second embodiment includes two expandable frame parts 80 that connect the proximal end portion 10' and the distal end portion 12. One end of each of the expandable frame parts 80 is connected to the proximal end portion 10', and the other end of each of the expandable frame parts 80 is connected to the distal end portion 12 or constitutes the distal end portion 12. Similarly to the first embodiment, a string 11 comes out from the proximal end portion 10' of the embryo transfer device 2, and the embryo transfer device 2 is formed of materials the same as or similar to those in the first embodiment.

In a no-load state, each of the expandable frame parts 80 includes: a first bent section 81 that is curved in the direction away from an X axis from the one end toward the other end; and a second bent section 82 that is disposed on the other end side with respect to the first bent section 81 and that is bent in the direction approaching the X axis from the one end toward the other end. The X axis is an axis that passes through the proximal end portion 10' and the distal end portion 12.

Furthermore, each of the expandable frame parts 80 includes: a first connecting section 83 that connects the first bent section 81 and the second bent section 82; and a second connecting section 84 that connects the second bent section 82 and the distal end portion 12. Similarly to the first embodiment, each of the expandable frame parts 80 may have various shapes.

Each of the expandable frame parts 80 in the second embodiment also includes: a widthwise outer section 18; and a support section (the one end side of the second bent section 82, the first connecting section 83, the first bent section 81, etc.) that supports the proximal end portion 10', serving as an embryo accommodation portion, in relation to the widthwise outer section 18. There are cases where, similarly to the first embodiment, the support section includes a Z-direction sloped section.

The two expandable frame parts 80, when released from the tube P, move in the Y direction due to the restoring force of the elastic deformation so that at least intermediate sections thereof in the length direction go apart from each other and go away from the X axis, and are expanded as a result of this movement. This expansion operation is similar to that in the first embodiment.

With the two expandable frame parts 80, similarly to the first embodiment, the expandable frame parts 80 serve as a movement resistance for the proximal end portion 10' in the uterine cavity 210, whereby an advantage is afforded in that the state in which the embryo in the proximal end portion 10' can come into contact with or is in contact with the endometrium is maintained.

Also in the second embodiment, the widthwise outer sections 18 are disposed at a position more distant from the X axis than the proximal end portion 10' serving as an embryo accommodation portion. Thus, an advantage is afforded in that the proximal end portion 10' serving as an embryo accommodation portion is hardly disposed on the outside in the width direction of the uterine cavity 210. Furthermore, with this configuration, an advantage is afforded in that the embryo is prevented or inhibited from being disposed on the oviduct side.

Furthermore, also in the second embodiment, the pair of expandable frame parts 80, the distal end portion 12, and the proximal end portion 10' are disposed so as to span the both side walls, the deep side, and the entry side in the uterine cavity 210, and it can be said that the ring-shaped arrangement mentioned earlier is realized even with the range of the pair of expandable frame parts 80 alone. In order to realize the stabilization of the position of the proximal end portion 10' serving as an embryo accommodation portion in the uterine cavity 210 and a reduction in the force that is applied from the embryo transfer device 2 to the endometrium, the pair of expandable frame parts 80 should preferably occupy not less than 70%, more preferably not less than 80%, and further preferably not less than 90% of the length in the circumferential direction of the ring shape.

Also in the second embodiment, an advantage is afforded in that the position of the proximal end portion 10' in the Y direction tends to be stable compared with the case of supporting the proximal end portion 10' serving as an embryo accommodation portion is supported with a thin rubber rod that extends from the proximal end portion 10' along the X axis and that can be inserted into the tube P.

Note that there may be a case where the embryo transfer device 1 or 2 includes three or more expandable frame parts. For example, the embodiment in Fig. 2 may be configured such that one expandable frame part 30 in the Y direction is formed of two expandable frame parts disposed so as to overlap each other in the Z direction and such that the other expandable frame part 30 in the Y direction is formed of two expandable frame parts disposed so as to overlap each other in the Z direction. In this case, the embryo transfer device 1 includes four expandable frame parts.

Furthermore, the embryo transfer device 1 or 2 may be configured such that another member such as a thin rubber frame, a string, or a plastic frame is disposed between the pair of expandable frame parts 30, 80 in Figs. 2, 17, and etc., one end of the member is connected to the proximal end portion 10 or 10', and the other end of the member is connected to the embryo accommodation portion 20 or the distal end portion 12. As described above, there may be cases where additional members, structures, etc. not shown in each of the embodiments described above are added as appropriate.

Furthermore, it is also possible to configure the distal end portion 12 of the embryo transfer device 2 shown in Fig. 17 as the embryo accommodation portion 20 of the first embodiment. In this case, two embryo accommodation portions are provided in the embryo transfer device 2. Alternatively, it is also conceivable to provide each of the expandable frame parts 80 of the embryo transfer device 2 with one embryo accommodation portion. In this case, a plurality of embryo accommodation portions are provided in the embryo transfer device 2. It is also conceivable to apply a similar modification to the embryo transfer device 1 shown in Fig. 2, etc.

Furthermore, the embryo transfer device 1 or 2 may be configured as shown in Fig. 14 such that a cover part 11a covering a prescribed length range of the string 11 is provided and such that the cover part 11a is formed of the same rubber material as the proximal end portion 10, etc. of the embryo transfer device 1. The prescribed length range is, for example, not less than 30%, not less than 50%, or not less than 79% of the length of the string 11. Furthermore, one end of the cover part 11a is connected to an end or a portion of the proximal end portion 10.

In one example, the cover part 11a is formed by using a mold simultaneously when the proximal end portion 10 is molded. For example, in the state where the string 11 is disposed in the mold, the mold is filled with a rubber material, whereby the string 11 is covered with the cover part 11a in such a manner that one end of the cover part 11a is connected to the proximal end portion 10. This configuration is advantageous in attaining the advantage that it is possible to reduce damage to the uterine cervix 220, the uterine cavity 210, the body of the patient, the mind of the patient, etc. at the time of withdrawal. Furthermore, this configuration may also lead to a reduction in the possibility that germs enter the body through the gaps between the fibers of the string 11. For the purpose of a reduction in this possibility, it is also possible to use a monofilament fiber as the string 11 in each of the embodiments described above. Due to the molding described above, there may be cases where a portion of the string 11 is exposed from a side surface 11b of the cover part 11a.

In each of the embodiments described above, one end of each of the frame parts 30, 50, 50', 50", 60, 70, and 80 is the proximal end and the other end thereof is the distal end, and each of the frame parts 30, 50, 50', 50", 60, 70, and 80 is connected to the embryo accommodation portion 20 or 21. Furthermore, when disposed in the tube P, the frame parts 30, 50, 50', 50", 60, 70, and 80 enter an accommodated state following the shape of the interior (hollow section) of the tube P. Meanwhile, when released from the tube P and disposed in the uterine cavity 210, the frame parts 30, 50, 50', 50", 60, 70, and 80 are expanded in the direction mainly along the Y axis. Furthermore, the embryo accommodation portion 20 or 21 is opened in the direction along the Z axis.

With each of the embodiments described above, compared with the case where the embryo accommodation portion 20 or 21 is supported with a thin rubber rod that extends from the embryo accommodation portion 20 or 21 along the X axis and that can be inserted into the tube P, an advantage is afforded in that the position of the embryo accommodation portion 20 or 21 in the Y direction tends to be stable, and thus the state in which the embryo can come into contact with or is in contact with the endometrium in the uterine cavity 210 tends to be maintained.

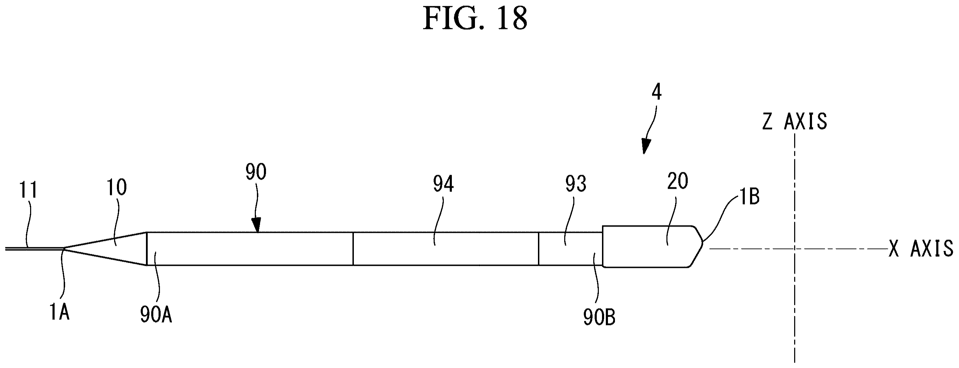

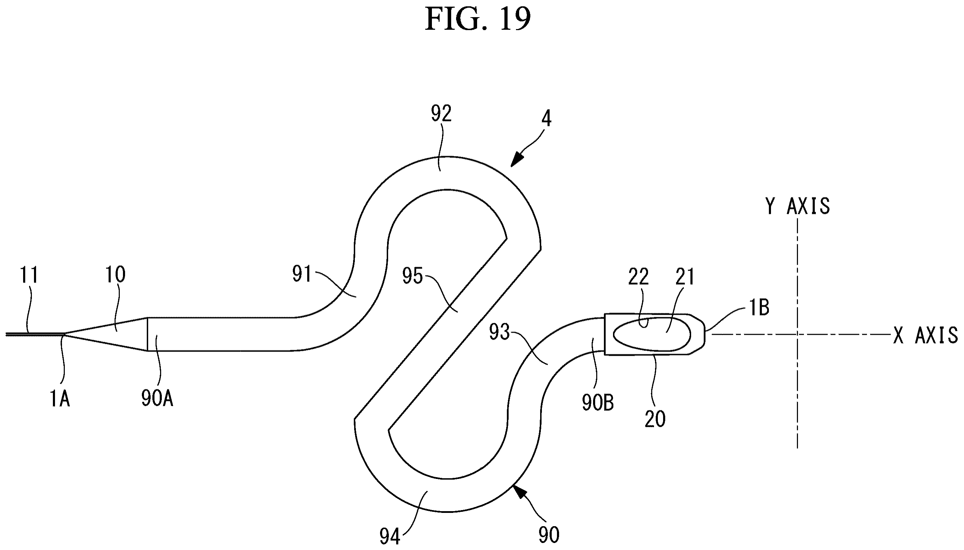

As shown in Figs. 18 and 19, it is also possible to manufacture an embryo transfer device 4 wherein a proximal end 90A of a frame part 90 is connected to a proximal end portion 10 similar to that in the first embodiment and a distal end 90B of the frame part 90 is connected to an embryo accommodation portion 20 similar to that in the first embodiment.

Similarly to the first embodiment, the proximal end portion 10 constitutes the proximal end 1A of the embryo transfer device 4, and the embryo accommodation portion 20 constitutes the distal end 1B of the embryo transfer device 4.

The shape of the embryo transfer device 4 in the no-load state will be described below. The X axis of the embryo transfer device 4 passes through the proximal end 90A and the distal end 90B of the frame part 90. Furthermore, the frame part 90 includes: a first bent section 91 that is curved in the direction away from the X axis from the proximal end 90A toward the distal end 90B; and a second bent section 92 that is disposed on the distal end 90B side with respect to the first bent section 91 and that is bent in the direction approaching the X axis from the proximal end 90A toward the distal end 90B.

Furthermore, the frame part 90 includes a third bent section 93 that is disposed on the distal end 90B side with respect to the second bent section 92 and that is bent in the direction away from the X axis from the distal end 90B toward the proximal end 90A. Furthermore, the frame part 90 includes a fourth bent section 94 that is disposed on the distal end 90B side with respect to the second bent section 92, that is disposed on the proximal end 90A side with respect to the third bent section 93, and that is bent in the direction approaching the X axis from the proximal end 90A toward the distal end 90.

Although the second bent section 92 and the fourth bent section 94 are connected to each other via a connecting section 95, frame parts 90 having other shapes may be adopted. In one example, the frame part 90 is formed of the same material as in the first and second embodiments and is integrally molded with the proximal end portion 10 and the embryo accommodation portion 20 by using a mold.

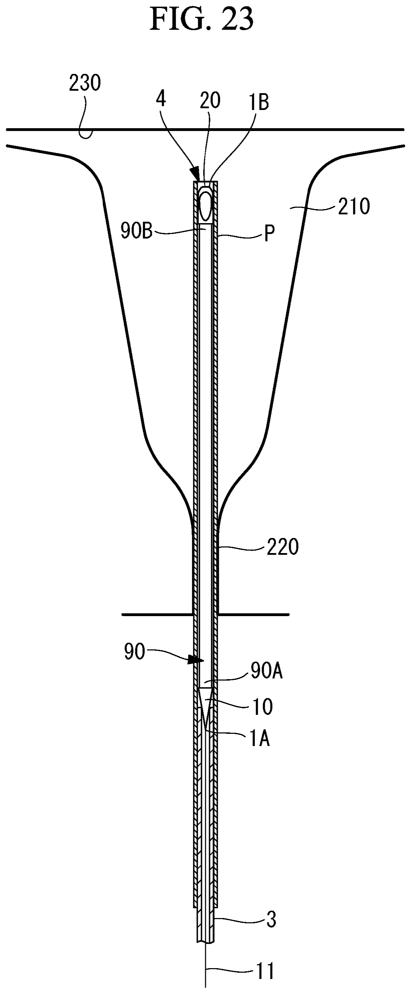

When disposed in the tube P, as shown in Fig. 23, the frame part 90 enters an accommodated state following the shape of the interior (hollow section) of the tube P. At this time, the curvature of each of the bent sections 91, 92, 93, and 94 becomes loose, or each of the bent sections 91, 92, 93, and 94 enters a state of being extended substantially linearly.

Furthermore, when released from the tube P and disposed in the uterine cavity 210, similarly to the first and second embodiments, the frame part 90 is expanded mainly in the direction along the Y axis.

Accordingly, an advantage is afforded in that the position of the embryo accommodation portion 20 in the Y direction tends to be stable compared with the case where the embryo accommodation portion 20 is supported with a thin rubber rod that extends from the embryo accommodation portion 20 along the X axis and that can be inserted into the tube P. Furthermore, the state in which the embryo can come into contact with or is in contact with the endometrium in the uterine cavity 210 tends to be maintained.

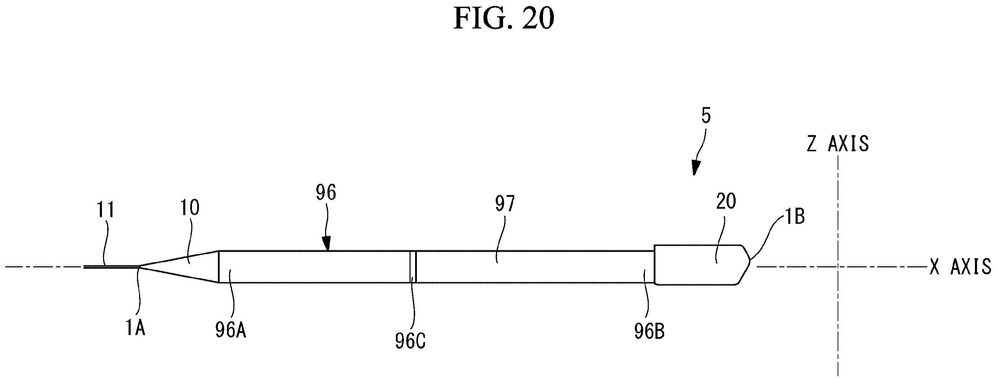

Furthermore, as shown in Figs. 20 to 22, it is also possible to manufacture an embryo transfer device 5 wherein a proximal end 96A of a frame part 96 is connected to a proximal end portion 10 similar to that in the first embodiment, and a distal end 96B of the frame part 96 is connected to an embryo accommodation portion 20 similar to that in the first embodiment.

Similarly to the first embodiment, the proximal end portion constitutes the proximal end 1A of the embryo transfer device 5, and the embryo accommodation portion 20 constitutes the distal end 1B of the embryo transfer device 5.

The shape of the embryo transfer device 5 in the no-load state will be described below. The X axis of the embryo transfer device 5 passes through the proximal end 96A and the distal end 96B of the frame part 96. In one example, the frame part 96 has a cylindrical shape, a prism shape, or the like that is elongated along the X axis from the proximal end 96A to the distal end 96B.

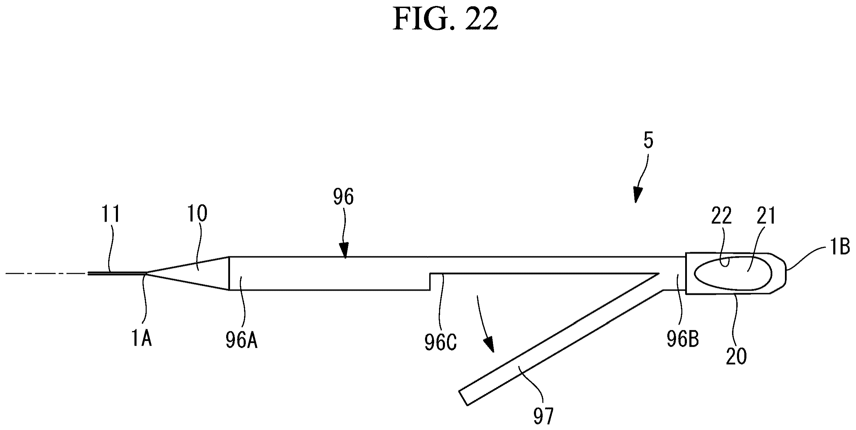

A recess 96C is provided at a portion in the length direction of the frame part 96, and an expandable part 97 is provided in the recess 96C. In one example, the frame part 96 and the expandable part 97 are formed of the same material as in the first and second embodiments and are integrally molded by using a mold. One end of the expandable part 97 is fixed to the frame part 96 or the embryo accommodation portion 20, and the other end of the expandable part 97 is expanded in the Y direction so as to go away from the X axis in the no-load state (Fig. 22).

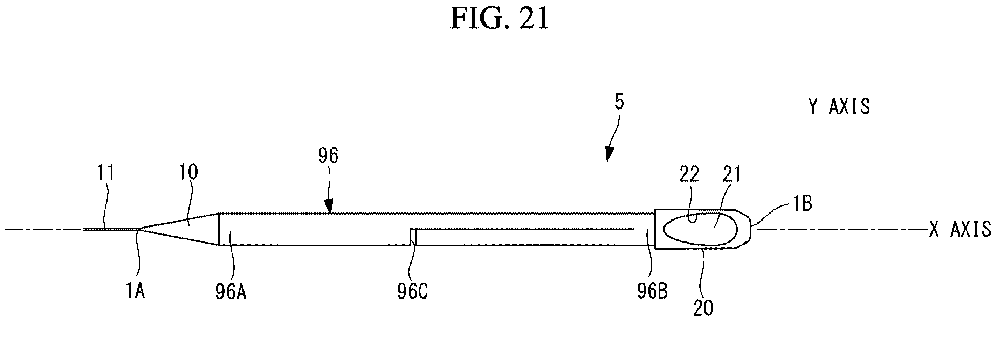

When disposed in the tube P, the frame part 96 and the expandable part 97 enter an accommodated state following the shape of the interior (hollow section) of the tube P. At this time, the frame part 96 and the expandable part 97 enter a state of being close to each other, as shown in Fig. 21.

Furthermore, when released from the tube P and disposed in the uterine cavity 210, similarly to the first and second embodiments, the expandable part 97 is expanded mainly in the direction along the Y axis.

Accordingly, an advantage is afforded in that the position of the embryo accommodation portion 20 in the Y direction tends to be stable compared with the case where the embryo accommodation portion 20 is supported with a thin rubber rod that extends from the embryo accommodation portion 20 along the X axis and that can be inserted into the tube P. Furthermore, the state in which the embryo can come into contact with or is in contact with the endometrium tends to be maintained.

Note that it is possible to change the shapes or structures of the frame part 96 or the expandable part 97. For example, depending on conditions, there may be cases where the configuration in which the other end of the expandable part 97 is fixed to the frame part 96 and the other portion of the expandable part 97 is expanded in the Y direction is adopted.

Although embodiments of the present disclosure have been described above in detail, the present disclosure is not limited to the individual embodiments described above. These embodiments allow various kinds of additions, substitutions, modifications, partial omissions, etc. within the scope not departing from the gist of the present invention or within the scope not departing from the idea and spirit of the present invention as derived from the content recited in the claims and equivalents thereof. For example, in the embodiments described above, it is possible to change the order of individual operations, to change the order of individual processing steps, to omit or add some operations depending on conditions, and to omit or add some processing steps depending on conditions, without having to adhere to the above examples. Furthermore, this is also applicable to cases where numerals or mathematical formulas are used in the descriptions of the above embodiments.

### {Reference Signs List}

1, 2, 4, 5 Embryo transfer device
1A, 2A, 90A, 96A Proximal end
1B, 2B, 90B, 96B Distal end
10, 10' Proximal end portion
11 String
15, 15' Distal-direction inclined section
16 Proximal-direction inclined section
17, 17' Z-direction sloped section
18 Widthwise outer section
20 Embryo accommodation portion
30, 50, 50', 50", 60, 70, 80 Expandable frame part
31 First bent section
32 Second bent section
33 Third bent section
34 Fourth bent section
41 First connecting section
42, 42' Second connecting section
43 Third connecting section
61 First bent section
62 Second bent section
63 Third bent section
90, 96 Frame part
97 Expandable part

## Claims

1. An embryo transfer device that has a proximal end and a distal end and that is inserted into a uterine cavity from a side of the distal end by using a tube inserted into a uterine cervix, the embryo transfer device comprising:
a proximal end portion forming a side of the proximal end;
an embryo accommodation portion that is disposed on the side of the distal end and that allows an embryo to be accommodated therein; and
two or more expandable frame parts that connect the proximal end portion and the embryo accommodation portion with each other,
wherein the two or more expandable frame parts are configured to become a state of being close to each other due to elastic deformation when disposed in the tube, and to expand in a direction along a Y axis due to a restoring force of the elastic deformation when released from the tube to be disposed in the uterine cavity, the Y axis being orthogonal to an X axis passing through the proximal end portion and the embryo accommodation portion.

2. The embryo transfer device according to claim 1, wherein the embryo accommodation portion is opened in a direction along a Z axis that is orthogonal to the X axis and the Y axis.

3. The embryo transfer device according to claim 1 or 2, wherein each of the expandable frame parts is made of a material having a hardness equal to or less than 90 points when measured with a type-A durometer.

4. The embryo transfer device according to claim 2,
wherein each of the expandable frame parts has one end connected to the proximal end portion and the other end connected to the embryo accommodation portion, and
wherein each of the expandable frame parts in a no-load state, in which no load is applied to the expandable frame part, at least includes:
a first bent section that is bent in a direction away from the X axis from the one end toward the other end;
a second bent section that is disposed at a side of the other end with respect to the first bent section and that is bent in a direction approaching the X axis from the one end toward the other end; and
a third bent section that is disposed at a side of the other end with respect to the second bent section and that is bent in a direction away from the X axis from the other end toward the one end.

5. The embryo transfer device according to claim 4, wherein each of the expandable frame parts includes a fourth bent section that is disposed at the side of the other end with respect to the second bent section, that is disposed at the side of the one end with respect to the third bent section, and that is bent in a direction approaching the X axis from the one end toward the other end.

6. The embryo transfer device according to claim 2,
wherein each of the expandable frame parts has one end connected to the proximal end portion and the other end connected to the embryo accommodation portion, and
wherein each of the expandable frame parts in a no-load state, in which no load is applied to the expandable frame part, at least includes:
a first convex curved section that is curved so as to have a shape convex in a direction away from the X axis; and
a second convex curved section that is disposed at a side of the other end with respect to the first convex curved section and that is curved so as to have a shape convex in a direction away from the X axis.

7. The embryo transfer device according to claim 4 or 5, wherein, in the no-load state, a distal-end-side angle of the angles, which are made by the X axis and an extending direction of a portion of each of the expandable frame parts extending from the third bent section to the side of the one end, is less than 90°.

8. The embryo transfer device according to claim 4 or 5, wherein, in the no-load state, each of the expandable frame parts includes a distal-direction inclined section that is inclined to the side of the distal end in a portion extending from the third bent section to the side of the one end.

9. The embryo transfer device according to claim 4 or 5, wherein a Z-direction sloped section, which is sloped in a direction along the Z axis in the no-load state, is provided in a range from the other end to the second bent section in each of the expandable frame parts.

10. The embryo transfer device according to any one of claims 1, 2, 4, and 5, wherein a discharge preventing section for preventing the embryo transfer device from being discharged from the uterine cavity is provided in the proximal end portion or the two or more expandable frame parts.

11. An embryo transfer device that has a proximal end and a distal end and that is inserted into a uterine cavity from a side of the distal end by using a tube inserted into a uterine cervix, the embryo transfer device comprising:
an embryo accommodation portion that allows an embryo to be accommodated therein; and
two or more expandable frame parts,
wherein the two or more expandable frame parts become a state of being close to each other due to elastic deformation when disposed in the tube, and expand in a direction along a Y axis due to a restoring force of the elastic deformation when released from the tube to be disposed in the uterine cavity, the Y axis being orthogonal to an X axis passing through the proximal end and the distal end, and
wherein the embryo accommodation portion is opened in a direction along a Z axis that is orthogonal to the X axis and the Y axis.

12. The embryo transfer device according to claim 11, wherein the two or more expandable frame parts, as a result of expansion, set a position of the embryo accommodation portion at a center side in a width direction of the uterine cavity.

13. The embryo transfer device according to any one of claims 1, 2, 11, and 12,
wherein at least two of the two or more expandable frame parts include:
widthwise outer sections that constitute portions of the embryo transfer device at both outer sides in a direction along the Y axis; and
support sections that constitute portions between the widthwise outer sections and the embryo accommodation portion and that support the embryo accommodation portion relative to the widthwise outer sections.

14. The embryo transfer device according to claim 13, wherein the support sections extend mainly in a direction along the Y axis.

15. An embryo transfer device that is inserted into a uterine cavity by using a tube inserted into a uterine cervix, the embryo transfer device comprising:
an embryo accommodation portion that allows an embryo to be accommodated therein; and
a frame part that has a proximal end and a distal end and that is connected to the embryo accommodation portion,
wherein an expandable part having one end fixed to the frame part or the frame part becomes an accommodated state following a shape of an interior of the tube when disposed in the tube, and is expanded mainly in a direction along a Y axis when released from the tube to be disposed in the uterine cavity, the direction along the Y axis being a direction orthogonal to an X axis passing through the proximal end and the distal end, and
wherein the embryo accommodation portion is opened in a direction along a Z axis that is orthogonal to the X axis and the Y axis.
